# EUROPEAN PATENT APPLICATION

(11) **EP 1 886 665 A1**
(43) Date of publication of application: **13.02.2008**
(21) Application number: 06118268.9
(22) Date of filing: 01.08.2006
(51) Int. Cl.: A61K 9/00, A61K 9/20

(54) **Gastro retentive delivery system**

(71) Applicant: Boehringer Ingelheim Pharma GmbH & Co. KG, 55216 Ingelheim am Rhein (DE)
(72) Inventor: Eisenreich, Wolfram, 89081 Ulm (DE); Friedl, Thomas, 88416 Ochsenhausen (DE); Haertter, Sebastian, 88447 Warthausen (DE)
(74) Representative: Hammann, Heinz

(57) **Abstract**

The present invention is directed to pharmaceutical composition for the manufacture of a gastro retentive drug delivery system comprising a pharmaceutical formulation and an application condition of the same.

## Description

### FIELD OF THE INVENTION

The present invention is directed to pharmaceutical composition for the manufacture of a gastro retentive drug delivery system comprising a pharmaceutical formulation and a defined application condition of the same.

### BACKGROUND OF THE INVENTION

For more than 20 years, development of gastric retained dosage forms was attempted resulting in only a limited number of promising technologies and products on the market.

Gastric retention of more than 6 hours still poses a considerable challenge to the pharmaceutical art.

Subject of the present invention is the development of a gastro retentive drug delivery system providing an extended residence time of the dosage form in the stomach of preferably more than 4 hours. Such a system is useful to improve the bioavailability and the duration of action of drugs.

### DESCRIPTION OF THE INVENTION

The invention relates to a gastro retentive drug delivery system enabling an extended residence time in the stomach. Typically, the gastric emptying time is in fasted state in the range from 0-2 hours and in the fed state from 4-6 hours. Purpose of the present invention is to describe a drug delivery system that stays in the stomach for at least 4, preferably 6 hours.

Surprisingly it was found that a sustained release formulation shows stomach residence times of more than 4 hours if taken in fed state. According to the present invention such a combination of a pharmaceutical formulation and a defined application scheme provides a new drug delivery platform technology with an interesting gastro retentive profile for many drugs.

The pharmaceutical formulation which is part of gastro retentive drug delivery system according to the invention combines retarding and mucoadhesive properties. According to the invention the formulation comprises either one polymer having both effects or two polymers, one providing the mucoadhesive property, the other one providing a retarding effect. Preferably the invention relates to a gastro retentive tablet formulation, wherein the matrix comprises at least two water swelling polymers and wherein at least one of the at least two polymers is an anionic polymer.

To provide a mucoadhesive effect, the invention makes use of "retarding polymers with mucoadhesive properties", preferably anionic polymers. Without limitation, such swelling retarding polymers may be selected from the group of carboxyalkylcelluloses such as carmellose sodium or carmellose calcium, chondroitin sulfate, acrylic acid polymerisate, pectin, alginates, carrageenans, chitin derivates such as chitosan, preferably acrylic acid polymerisate or chitosan. Among the preferred anionic polymer is an optionally crosslinked acrylic acid polymer. As acrylic acid polymerisate one may use one of the carbomer or carbopol® series, having high molecular weights. Particularly preferred are for example carbomer 941 (carbopol® 71 G, carbopol® 971) and carbomer 934 (carbopol® 974). The content of the optionally crosslinked acrylic acid polymer in the matrix is from about 0.1 wt.-% to about 40 wt.-% and preferably from about 0.1 wt.-% to about 20 wt.-%.

To provide a retarding or an increased retarding effect, the formulation according to the invention additionally may comprise a "swelling retarding polymer", a water swelling substantially neutral polymer. Without limitation, such swelling retarding polymers may be selected from the group of alkylcelluloses, such as, methylcellulose; hydroxyalkylcelluloses, for example, hydroxymethylcellulose (HPMC), hydroxyethylcellulose, hydroxypropylcellulose and hydroxybutylcellulose; hydroxyalkyl alkylcelluloses, such as, hydroxyethyl methylcellulose and hydroxypropyl methylcellulose; other natural, semi-synthetic, or synthetic di-, oligo- and polysaccharides such as galactomannans, tragacanth, agar, guar gum, and polyfructans; ammonio methacrylate copolymers; polyvinylalcohol; polyvinylpyrrolidone, copolymers of polyvinylpyrrolidone with vinyl acetate; combinations of polyvinylalcohol and polyvinylpyrrolidone; polyalkylene oxides such as polyethylene oxide and polypropylene oxide; copolymers of ethylene oxide and propylene oxide; preferably polyethylene oxide and cellulose ether derivatives such as hydroxypropyl methylcellulose and hydroxypropylcellulose, most preferred hydroxypropyl methylcellulose.

Such neutral polymer swells upon contact with aqueous fluid following administration, resulting in a viscous, drug release regulating gellayer. The viscosity of the polymer preferably ranges from 50 to 100,000 mPa.s (apparent viscosity of a 2% aqueous solution at 20°C.).

Preferably, the amount of water swelling polymer in the present formulation ranges from about 10 to about 80% by weight.

Among the substantially neutral polymers hydroxypropylcellulose and hydroxypropyl methylcellulose are preferred.

Different viscosity grades of hydroxypropylcellulose and hydroxypropyl methylcellulose are commercially available. Hydroxypropyl methylcellulose (HPMC) preferably used in the present invention has a viscosity grade ranging from about 50 mPa.s to about 100,000 mPa.s, in particular ranging from about 75 mPa.s to about 20,000 mPa.s and most in particular a viscosity grade of about 100 mPa.s to about 15,000 mPa.s (apparent viscosity of a 2% aqueous solution at 20°C.), e.g. hypromellose 2910, 2208 or 2206 (DOW, Antwerp, Belgium). HPMC type 2208 contains 19-24% by weight methoxy and 4-12% by weight hydroxypropoxy substituents.

Hydroxypropylcellulose having a viscosity higher than 1,500 mPa.s (apparent viscosity of a 1% aqueous solution at 20°C) is preferred, in particular hydroxypropylcellulose having a viscosity in the range from about 1500 to about 3000 mPa.s, preferably from 4000 to 6500 mPa.s (2% aqueous solutions), e.g. the Klucel series such as Klucel M (Hercules, Wilmington, USA).

According to a preferred embodiment of the present invention the matrix of a gastro retentive tablet formulation comprises or essentially consists of hydroxypropyl methylcellulose, such as hypromellose, and further excipients. The amount of hydroxypropyl methylcellulose is preferably in the range from 10 to 80%, particularly preferred from 15 to 65% most preferred from 20 to 50% by weight. The amount of further excipients is preferably in the range from 80 to 25%, particularly preferred from 75 to 35%, most preferred from 65 to 45% by weight.

Such systems with mucoadhesive and retarding properties are useful to extend the gastric residence time by adhering them to the gastric mucous membrane. Even though some of the mucoadhesive polymers are effective at producing bioadhesion, it is very difficult to maintain a residence time over several hours because of the rapid turnover of mucus in the gastrointestinal tract.

In addition, when using a combination of a neutral and anionic polymer, the ratio of said polymers also may influence the gastro retentive profile of the preparation. Accordingly, such combination facilitates control of the gastro retentive profile of the preparation at will and it will be perspicuous for the skilled person in the art, that the gastro retentive profile may be adjusted via the ratio of said polymers, which is another benefit of the present invention.

According to a preferred embodiment of the present invention a tablet formulation is provided having a matrix that comprises or essentially consists of hydroxypropyl methylcellulose, acrylic acid polymerisate and further excipients. The amount of hydroxypropyl methylcellulose is preferably in the range from 10 to 80%, particularly preferred from 15 to 65%, most preferred from 20 to 50% by weight. The amount of acrylic acid polymerisate is preferably as above-mentioned. The amount of additional excipients is preferably in the range from 80 to 25% particularly preferred from 75 to 35%, most preferred from 65 to 45% by weight. Optionally carboxymethylcellulose sodium may additionally be present preferably in the range from 5 to 50%, particularly preferred from 10 to 40%, most preferred from 15 to 30% by weight.

The formulation of the present invention optionally comprises an active ingredient. Such active ingredient may
- show pH-dependent solubility and/or
- show a limited absorption window in the gastrointestinal tract and/or
- be intended for local treatment in the stomach or the small intestine and/or
- show low stability in intestinal fluids and/or
- degrades by enzymes/bacteria present in the intestine.

However, the formulation also may be a placebo, meaning that the formulation does not comprise an active ingredient.

The formulation according to the invention optionally comprise further excipients, i.e. pharmaceutically acceptable formulating agents, in order to promote the manufacture, compressibility, appearance and taste of the preparation. These formulating agents comprise, for example, diluents or fillers, glidants, binding agents, granulating agents, anti-caking agents, lubricants, flavors, dyes and preservatives. Other conventional excipients known in the art can also be included.

The filler may be selected from soluble fillers, for example, sucrose, lactose, in particular lactose monohydrate, trehalose, maltose, mannitol and sorbitol. Different grades of lactose can be used. One type of lactose preferably used in the present invention is lactose monohydrate 200 mesh (DMV, Veghel, The Netherlands). Another lactose monohydrate, lactose monohydrate of the type DCL 11 (DMV, Veghel, The Netherlands), can also preferably be used. The notation DCL refers to "Direct Compression Lactose". The number 11 is a reference number of the manufacturer. In case of a water soluble active ingredient, more preferably water insoluble fillers, such as starch and starch derivates preferably other than pregelatinized starch, e.g. corn starch, potato starch, rice starch or wheat starch, microcrystalline cellulose, dibasic calcium phosphate dihydrate and anhydrous dibasic calcium phosphate, preferably corn starch, can be used in addition or instead of the water soluble fillers. The total weight percentage of filler ranges between about 5% and about 75% by weight.

A glidant can be used to improve powder flow properties prior to and during tableting and to reduce caking. Suitable glidants include colloidal silicon dioxide, talc, magnesium trisilicate, powdered cellulose, talc, tribasic calcium phosphate and the like. Colloidal silicon dioxide is preferably included as a glidant in an amount up to about 2%, preferably about 0.2% to about 0.8%, by weight of the tablet.

A lubricant can be used to enhance release of a tablet from apparatus on which it is formed, for example by preventing adherence to the face of an upper punch ("picking") or lower punch ("sticking"). Suitable lubricants include magnesium stearate, calcium stearate, canola oil, glyceryl palmitostearate, hydrogenated vegetable oil, magnesium oxide, mineral oil, poloxamer, polyethylene glycol, polyvinyl alcohol, sodium benzoate, sodium lauryl sulfate, sodium stearyl fumarate, stearic acid, zinc stearate and the like. In one embodiment, magnesium stearate is included as a lubricant in an amount of about 0.1% to about 1.5%, preferably about 0.3% to about 1%, by weight of the tablet.

Among the optional formulating agents that further may be comprised in the matrix formulation there may be mentioned agents such as polyvidone; copovidone; starch; acacia; gelatin; seaweed derivatives, e.g. alginic acid, sodium and calcium alginate; cellulose, preferably microcrystalline cellulose, cellulose derivatives, e.g. ethylcellulose, hydroxypropylcellulose, hydroxypropyl methylcellulose, having useful dry or wet binding and granulating properties; and antiadherents such as talc and magnesium stearate.

The expression "consisting essentially" is understood in the sense that it does not in principle exclude the presence, in addition to the mandatory components mentioned, of other components, the presence of which does not affect the essential nature of the formulation.

In a preferred embodiment of the present invention the tablet formulation with gastro retentive properties is provided preferably having the following composition:

| | |
|---|---|
| active ingredient | 0.01 - 50 % by weight |
| swelling retarding polymer | 10 to 80 % by weight, |
| | preferably 20 - 50 % by weight |
| retarding polymers with mucoadhesive properties | 0.1 - 40 % by weight, |
| | preferably 0.1 - 20 % by weight |

In another preferred embodiment of the present invention the formulation tablet with gastro retentive properties is provided preferably having the following composition:

| | |
|---|---|
| active ingredient | 0.05 to 5% by weight |
| water swelling polymer(s) | 10 to 75% by weight |
| acrylic acid polymerisate | 0 to 25% by weight |
| optional further excipient(s) | ad 100% by weight. |

Therefore, a particularly preferred tablet formulation according to the invention consists of 0.1 to 35% by weight of active ingredient thereof;
25 to 65% by weight of hydroxypropyl methylcellulose;
0 to 40% by weight of carboxymethylcellulose sodium;
0 to 75% by weight of corn starch other than pregelatinized starch;
0 to 15% by weight of acrylic polymerisate, preferably carbomer 941;
0.5 to 50% by weight of excipients, preferably selected from the group consisting of colloidal silicon dioxide, magnesium stearate, lactose monohydrate, mannitol, microcrystalline cellulose, dibasic anhydrous calcium phosphate, hydroxyproylcellulose, povidone, copovidone, talc, macrogols, sodium dodecylsulfate, iron oxides and titanium dioxide.

According to the present invention starch, preferably other than pregelatinized starch, preferably corn starch if present, may impart several functions at the same time such as filler, glidant, and the like. However, it may be preferred to exclude starch completely from the tablet formulation according to the present invention, which may be replaced by one or more of the above-mentioned other excipient(s).

It is preferred that no coating is present on the tablet formulation according to the present invention. However, the tablet of the invention may comprise a nonfunctional coating. A nonfunctional coating can comprise a polymer component, for example HPMC, optionally with other ingredients, for example one or more plasticizers, colorants, etc. The term "nonfunctional" in the present context means having no substantial effect on release properties of the tablet, and the coating serves another useful purpose. For example, such a coating can impart a distinctive appearance to the tablet, provide protection against attrition during packaging and transportation, improve ease of swallowing, and/or have other benefits. A nonfunctional coating should be applied in an amount sufficient to provide complete coverage of the tablet. Typically an amount of about 1% to about 10%, more typically an amount of about 2% to about 5%, by weight of the tablet as a whole, is suitable.

The tablets of the present invention can be of any suitable size and shape, for example round, oval, polygonal or pillow-shaped, and optionally bear nonfunctional surface markings. According to the present invention it is preferred that the extended release tablets are white to off-white and of oval or round, biconvex, shape.

In a preferred embodiment of the invention a tablet is provided, having a size of at least 11 mm in case of a round shaped tablet and 15 x 7 mm in case of an oblong shaped tablet.

In another preferred embodiment of the invention a tablet is provided having weight in the range of 200 mg to 1500 mg, preferably 390 mg to 1000 mg, more preferably 400 mg to 750 mg and even more preferably of 500 - 750 mg.

Tablets of the invention can be packaged in a container, accompanied by package insert providing pertinent information such as, for example, dosage and administration information, contraindications, precautions, drug interactions and adverse reactions.

However, the aforementioned approach directed to the composition of the pharmaceutical formulation according to the invention alone will not provide the herein disclosed effect.

It is essential to apply the drug delivery system of the present invention in fed state, meaning after meal. This is as it has been observed that food, particularly fatty acids, prevents emptying of the stomach.

In the context of the present invention the term "fed state" means that patients take the drug at maximum 4 hours, preferably at maximum 3 hours, more preferably at maximum 2 hours, even more preferably at maximum 1 hour, even more preferably at maximum 30 minutes and most preferably just after meal. In an alternative embodiment the patients may take the drug delivery system while eating.

Furthermore, the present invention is preferably directed to a method of manufacturing the extended release tablet formulations via a direct compression process comprising the steps of
(1) producing an active ingredient trituration by preblending it with a portion of water swelling polymer(s) and/or further excipient(s) in a mixer;
(2) premixing the active ingredient trituration of step (1), the main portion of the water swelling polymer(s) and/or excipients in a mixer to obtain a pre-mixture;
(3) optionally dry screening the pre-mixture through a screen in order to segregate cohesive particles and to improve content uniformity;
(4) mixing the pre-mixture of step (2) or (3) in a mixer, optionally by adding remaining excipients to the mixture and continuing mixing; and
(5) tableting the final mixture by compressing it on a suitable tablet press to produce matrix tablets.

Also other processes can be applied to the manufacturing of tablets according to the invention, like conventional wet granulation and roller compaction. In case of wet granulation the active ingredient may be granulated with suitable fillers, like e.g. starches other than pregelatinized starch, microcrystalline cellulose, lactose monohydrate or anhydrous dibasic calcium phosphate, and wet binding agents, like e.g. hydroxypropyl methylcellulose, hydroxypropylcellulose, povidone, copovidone, and starch paste, leading to a active ingredient concentrate, which after drying and dry screening is mixed with the main fraction of gel forming excipients, like all the above described retarding principles.

In case of roller compaction, or in other words dry granulation, either a premix of active ingredient with part of the excipients used in the direct compression process, or the complete mixture containing all excipients, is processed through a conventional roller compactor to form ribbons, which are thereafter screened down to granules which are finally mixed with other excipients, like glidants, lubricants and antiadherents.

### BRIEF DESCRIPTION OF THE DRAWINGS

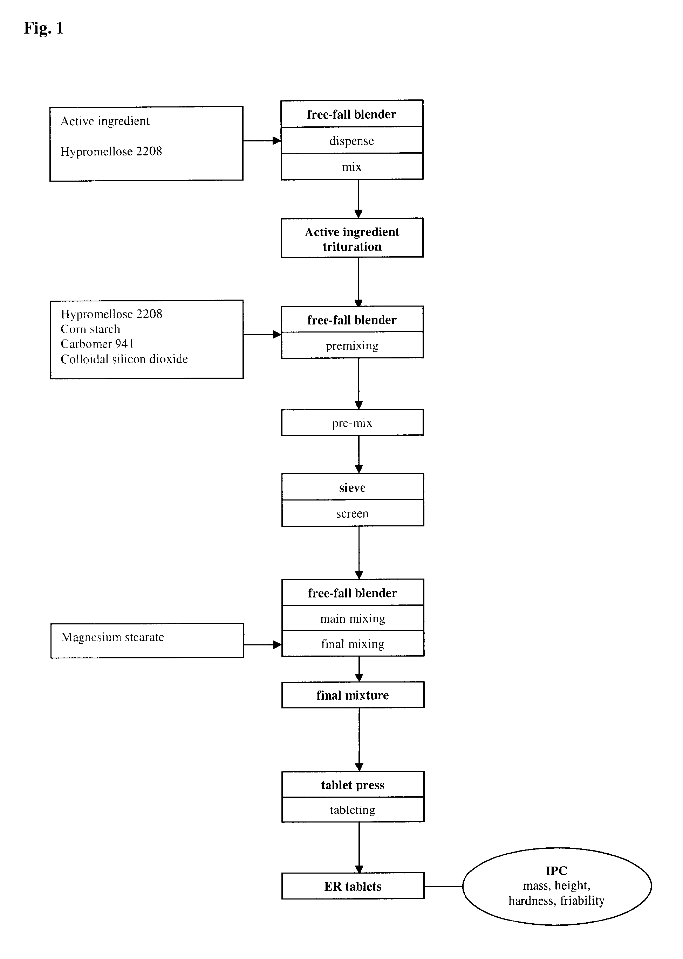

Figure 1 illustrates a preferred embodiment of the manufacturing process with reference to a flow diagram wherein the manufacture of the extended release tablets of Examples 4 and 5 are exemplarily shown. Figure 1 shows the detailed process steps and the in process controls performed.

Process step (1) is directed to the active ingredient trituration, where the active ingredient is preblended with a portion of the polymer, in this case hydroxypropyl methylcellulose, in a commonly known mixer. In the flow chart a Turbula free-fall mixer or blender is used. The mixing time is several minutes, in the present case preferably 10 min.

In process step (2) according to the flow chart a premixing is performed, wherein the active ingredient trituration and the main portion of the water swelling polymer(s) and excipients are premixed for several minutes to obtain a pre-mix. In the present case the main portion of hydroxypropyl methylcellulose (hypromellose), corn starch, carbomer 941 and colloidal silicon dioxide are premixed for 5 min. in the above-mentioned Turbula mixer or blender.

According to the following process step (3) a dry screening may optionally take place. The pre-mixture may be manually screened through a screen, for example a 0.8 mm mesh size screen, in order to segregate cohesive particles and to improve content uniformity.

In the subsequent process step (4) the main mixing step is performed according to which the components are mixed for several minutes, preferably 5 min. in the Turbula mixer after screening. Optionally further excipients may be added at this time, in the flow chart the component magnesium stearate is added to the main mixture, and further mixing for several minutes, e.g. 3 min., in the Turbula mixer is performed (final mixing) to obtain the final mixture.

Process step (5) of the process according to the present invention is the tableting. The final mixture is compressed on a suitable tablet press to produce, for example, oblong shaped matrix tablets (ER tablets = extended release tablets). In order to control and maintain the required quality the obtained matrix tablets are subjected to the following in-process controls: tablet mass, hardness, tablet height and friability.

The obtained tablets of the present invention may then be filled, for example, into High Density Polyethylene (HDPE) bottles. The bottles are closed tightly with screw caps and appropriately labelled, whereby all packaging and labelling activities are performed according to cGMP regulations. Alternatively, a blister type packaging can be used, e.g. using aluminium/aluminium foil blisters.

Furthermore, the tablets of the present invention may be manufactured via a direct compression, wet or dry granulation process.

### FORMULATION EXAMPLES

Placebo tablets were prepared with the following composition

| | |
|---|---|
| Hypromellose 2208 | 112.50 mg |
| Maize starch | 114.75 mg |
| Carbomer 941 | 15.00 mg |
| Iron oxide black | 5.00 mg |
| Colloidal anhydrous silica | 1.50 mg |
| Magnesium stearate | 1.25 mg |
| Total weight placebo tablet | 250.00 mg |
| ---- | |

| | |
|---|---|
| Hypromellose 2208 | 225.0 mg |
| Maize starch | 249.5 mg |
| Carbomer 941 | 15.0 mg |
| Iron oxide black | 5.0 mg |
| Colloidal anhydrous silica | 3.0 mg |
| Magnesium stearate | 2.5 mg |
| Total weight placebo tablet | 500.0 mg |
| ---- | |

| | |
|---|---|
| Hypromellose 2208 | 315.0 mg |
| Maize starch | 321.3 mg |
| Carbomer 941 | 42.0 mg |
| Iron oxide black | 14.0 mg |
| Colloidal anhydrous silica | 4.2 mg |
| Magnesium stearate | 3.5 mg |
| Total weight placebo tablet | 700.0 mg |

Further examples with active ingredients are

| | |
|---|---|
| Active ingredient | 0.750 mg |
| Hypromellose 2208 (Methocel K 15 M) | 157.500 mg |
| Corn starch | 183.700 mg |
| Carbomer 941 (Carbopol® 71 G) | 3.500 mg |
| Colloidal Silicon dioxide | 2.800 mg |
| Magnesium stearate | 1.750 mg |
| Total weight matrix tablet | 350.000 mg |
| ---- | |

| | |
|---|---|
| Active ingredient | 4.500 mg |
| Hypromellose 2208 (Methocel K 15 M) | 225.000 mg |
| Corn starch | 250.000 mg |
| Carbomer 941 (Carbopol® 71 G) | 15.000 mg |
| Colloidal Silicon dioxide | 3.000 mg |
| Magnesium stearate | 2.500 mg |
| Total weight matrix tablet | 500.000 mg |
| ---- | |

| | |
|---|---|
| Active ingredient | 0.750 mg |
| Hypromellose 2208 (Methocel K 15 M) | 157.500 mg |
| Corn starch | 174.600 mg |
| Carbomer 941 (Carbopol® 71G) | 14.000 mg |
| Colloidal Silicon dioxide | 1.400 mg |
| Magnesium stearate | 1.750 mg |
| Total weight matrix tablet | 350.000 mg |
| ---- | |

| | |
|---|---|
| Active ingredient | 1.500 mg |
| Hypromellose 2208 | 315.000 mg |
| Corn starch | 349.200 mg |
| Carbomer 941 | 28.000 mg |
| Colloidal Silicon dioxide | 2.800 mg |
| Magnesium stearate | 3.500 mg |
| Total weight matrix tablet | 700.000 mg |
| ---- | |

| | |
|---|---|
| Active ingredient | 0.750 mg |
| Hypromellose 2208 (Methocel K 100 M) | 180.000 mg |
| Corn starch | 215.400 mg |
| Colloidal silicon dioxide | 2.100 mg |
| Magnesium stearate | 1.750 mg |
| Total weight matrix tablet | 400.000 mg |
| ---- | |

| | |
|---|---|
| Active ingredient | 0.750 mg |
| Hypromellose 2208 (Methocel K 15 M) | 180.000 mg |
| Carboxymethylcellulose sodium | 100.000 mg |
| Lactose monohydrate (200 mesh) | 50.000 mg |
| Microcrystalline cellulose (grade PH 101) | 65.750 mg |
| Colloidal silicon dioxide | 1.500 mg |
| Magnesium stearate | 2.000 mg |
| Total weight matrix tablet | 400.000 mg |
| ---- | |

| | |
|---|---|
| Active ingredient | 100.000 mg |
| Hydroxypropylcellulose | 270.000 mg |
| Carboxymethylcellulose sodium | 60.000 mg |
| Lactose monohydrate (200 mesh) | 50.000 mg |
| Microcrystalline cellulose (grade PH 101) | 99.000 mg |
| Carbomer 941 (Carbopol® 71 G) | 6.000 mg |
| Colloidal silicon dioxide | 3.000 mg |
| Magnesium stearate | 12.000 mg |
| Total weight matrix tablet | 600.000 mg |
| ---- | |

| | |
|---|---|
| Active ingredient | 0.750 mg |
| Hypromellose 2208 (Methocel K 15 M) | 175.000 mg |
| Carboxymethylcellulose sodium | 87.500 mg |
| Lactose monohydrate (200 mesh) | 45.500 mg |
| Microcrystalline cellulose (grade PH 101) | 24.100 mg |
| Carbomer 941 (Carbopol® 71 G) | 14.000 mg |
| Colloidal silicon dioxide | 1.400 mg |
| Magnesium stearate | 1.750 mg |
| Total weight matrix tablet | 350.000 mg |
| ---- | |

| | |
|---|---|
| Active ingredient | 100.000 mg |
| Carbomer 941 (Carbopol® 71 G) | 100.000 mg |
| Lactose monohydrate (200 mesh) | 225.000 mg |
| Microcrystalline cellulose (grade PH 101) | 71.250 mg |
| Colloidal silicon dioxide | 1.250 mg |
| Magnesium stearate | 2.500 mg |
| Total weight matrix tablet | 500.000 mg |
| ---- | |

| | |
|---|---|
| Active ingredient | 0.750 mg |
| Carbomer 941 (Carbopol® 71 G) | 100.000 mg |
| Lactose monohydrate (200 mesh) | 300.000 mg |
| Microcrystalline cellulose (grade PH 101) | 93.000 mg |
| Colloidal silicon dioxide | 1.250 mg |
| Magnesium stearate | 5.000 mg |
| Total weight matrix tablet | 500.000 mg |
| ---- | |

| | |
|---|---|
| Active ingredient | 100.000 mg |
| Carbomer 941 (Carbopol® 71 G) | 100.000 mg |
| Lactose monohydrate (200 mesh) | 150.000 mg |
| Calcium Phosphate, dibasic dihydrate | 146.250 mg |
| Colloidal silicon dioxide | 1.250 mg |
| Magnesium stearate | 2.500 mg |
| Total weight matrix tablet | 500.000 mg |
| ---- | |

| | |
|---|---|
| Active ingredient | 0.750 mg |
| Carbomer 941 (Carbopol® 71 G) | 52.500 mg |
| Lactose monohydrate (200 mesh) | 140.000 mg |
| Calcium Phosphate, dibasic dihydrate | 153.600 mg |
| Colloidal silicon dioxide | 1.400 mg |
| Magnesium stearate | 1.750 mg |
| Total weight matrix tablet | 350.000 mg |
| ---- | |

| | |
|---|---|
| Active ingredient | 200.00 mg |
| Hypromellose 2208 (Methocel E50 LV) | 300.00 mg |
| Lactose monohydrate | 190.00 mg |
| Carbomer 941 (Carbopol® 71 G) | 37.500 mg |
| Colloidal silicon dioxide | 7.50 mg |
| Magnesium stearate | 15.00 mg |
| Total weight matrix tablet | 750.000 mg |
| ---- | |

| | |
|---|---|
| Active ingredient 100.00 | mg |
| Hypromellose 2910 100.00 (Methocel E50 LV) | mg |
| Microcrystalline cellulose 215.00 | mg |
| Methacrylic acid copolymers 25.00 | mg |
| Organic acid 50.00 | mg |
| Colloidal silicon dioxide 2.50 | mg |
| Magnesium stearate 7.50 | mg |
| Total weight matrix tablet 500.00 | mg |
| ---- | |

| | |
|---|---|
| Active ingredient | 100.00 mg |
| Hypromellose 2208 (Methocel K 100 LV) | 150.00 mg |
| Microcrystalline cellulose | 235.00 mg |
| Carbomer 941 (Carbopol® 71 G) | 5.00 mg |
| Colloidal silicon dioxide | 2.50 mg |
| Magnesium stearate | 7.50 mg |
| Total weight matrix tablet | 500.000 mg |

### GASTRO RETENTIVE EFFECT

The gastro retentive effect was proven with magnetically marked tablets of the aforementioned type (Placebo tablet of 250 mg and 500 mg weight with incorporation of Fe₃O₄-Magnetit.)

The tablets were applied to patients and the GI-transit was monitored via the magnetic properties of the tablets. The decline of aligned magnetic moment was correlated with in vivo disintegration.

In a randomised, open, four-way changeover magnetic marker monitoring study the gastrointestinal transit and in vivo disintegration process of two differently sized extended release matrix tablets containing Fe₃O₄ (E172) was evaluated at 8 healthy volunteers (4 males 4 females). The volunteers were given the magnetically marked tablets in fasted and fed state.
It was observed during routine in-vitro dissolution tests that the gastro retentive dosage form starts to swell after contact with fluids and that it starts to float on the top of the dissolution media. The swollen tablets have obviously a lower density than water.
The results showed that in particular tablets show a gastro retentive effect of more than 4 hours if taken in fed state. So it could be shown that the mean residence time in fed state was nine times longer than given without a meal.

Additionally it could be shown that while tablets with a weight of 250 mg already had a residence time in the stomach of more than 5 hours if taken after meal, larger tablets of 500 mg even showed a residence time of more than 8 hours when taken under the same conditions.

Accordingly, the results show that the large tablet stays significantly longer in the stomach.

## Claims

1. A pharmaceutical formulation which comprises 0.01 - 50 % by weight of active ingredient, 10 to 80 % by weight, preferably 20 - 50 % by weight of a swelling retarding polymer, 0.1 - 40 % by weight, preferably 0.1 - 20 % by weight of retarding polymers with mucoadhesive properties for the manufacture of a gastro retentive delivery system providing an extended residence time of the dosage form in the stomach of preferably more than 4 hours, **characterised in that** the pharmaceutical formulation is taken in fed state.
